(19) Europäisches Patentamt
European Patent Office
Office européen des brevets

(11) Veröffentlichungsnummer: **0 055 213**
**B1**

(12) # EUROPÄISCHE PATENTSCHRIFT

(45) Veröffentlichungstag der Patentschrift: **26.02.86**

(51) Int. Cl.[4]: **C 07 C 127/22**, C 07 C 87/60, A 01 N 47/34

(21) Anmeldenummer: **81810485.3**

(22) Anmeldetag: **07.12.81**

(54) **Phenylharnstoffe.**

(30) Priorität: **12.12.80 CH 9197/80**
**29.10.81 CH 6915/81**

(43) Veröffentlichungstag der Anmeldung: **30.06.82 Patentblatt 82/26**

(45) Bekanntmachung des Hinweises auf die Patenterteilung: **26.02.86 Patentblatt 86/9**

(84) Benannte Vertragsstaaten: **BE CH DE FR GB IT LI NL**

(56) Entgegenhaltungen:
**EP - A - 0 004 030**
**EP - A - 0 016 729**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Ehrenfreund, Josef, Dr., 79 Gleneagles Road, Flixton Manchester M31 2SA (GB)**

**EP 0 055 213 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER. STOCKHOLM 1986

**Beschreibung**

Die vorliegende Erfindung betrifft neue substituierte N-(p-Aminophenyl)-N'-benzoylharnstoffe, Verfahren zu ihrer Herstellung und ihre Verwendung in der Schädlingsbekämpfung.

Die erfindungsgemässen substituierten N-(p-Aminophenyl)-N'-benzoylharnstoffe haben die Formel

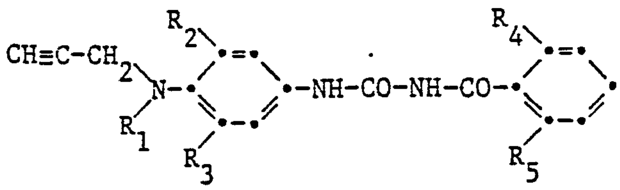
(I),

worin $R_1$ Wasserstoff, $C_1$—$C_4$-Akyl oder Propargyl, $R_2$ und $R_3$ je Wasserstoff oder Halogen, $R_4$ Methyl oder Halogen und $R_5$ Wasserstoff oder Halogen bedeuten.

Beispiele für Alkyl-Gruppen in der Definition $C_1$—$C_4$-Alkyl gamäss der Erfindung sind Methyl, Aethyl, n-Propyl, i-Propyl sowie die vier isomeren Butyl-Gruppen. Im Rahmen der Erfindung ist ferner unter Halogen Fluor, Chlor, Brom und Jod, vorzugsweise Fluor, Chlor und Brom, zu verstehen.

Wegen ihrer Wirkung als Schädlingsbekämpfungsmittel bevorzugt sind solche Verbindungen der Formel I, worin $R_1$ $C_1$—$C_4$-Alkyl oder Propargyl; $R_2$ und $R_3$ je Fluor, Chlor oder Brom; $R_4$ Fluor, Chlor, Brom oder Methyl und $R_5$ Wasserstoff, Fluor oder Chlor bedeuten. Dabei stehen im Vordergrund dieser Gruppe einerseits diejenigen Verbindungen, in denen $R_1$, $R_2$, $R_3$ und $R_5$ die angegebene Bedeutung haben und $R_4$ für Fluor oder Chlor steht und andererseits diejenigen Verbindungen, in denen $R_1$, $R_2$, $R_3$ und $R_4$ die angegebene Bedeutung haben und $R_5$ für Fluor oder Chlor steht.

Hervorzuheben sind ausserdem solche Verbindungen der Formel I, worin $R_1$ $C_1$—$C_4$-Alkyl oder Propargyl, $R_2$ und $R_3$ je Fluor, Chlor oder Brom, $R_4$ Fluor oder Chlor und $R_5$ Wasserstoff oder Fluor bedeuten. Im Vordergrund dieser Gruppe stehen einerseits diejenigen Verbindungen, in denen $R_1$, $R_2$ und $R_3$ die angegebene Bedeutung haben und $R_4$ und $R_5$ je für Fluor stehen, und andererseits diejenigen Verbindungen, in denen $R_1$, $R_2$ und $R_4$ die angegebene Bedeutung haben und $R_5$ für Wasserstoff steht.

Die Verbindungen der Formel I können nach an sich bekannten Verfahren hergestellt werden (vgl. u.a. die deutschen Offenlegungsschriften Nr. 2.123.236, 2.601.780). So kann man z.B. eine Verbindung der Formel I erhalten durch Umsetzung

a) einer Verbindung der Formel II

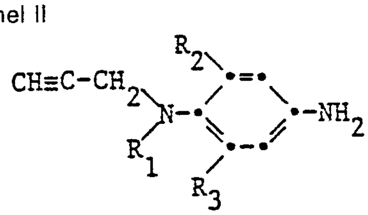
(II)

mit einer Verbindung der Formel III

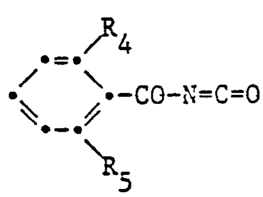
(III)

oder

b) einer Verbindung der Formel IV

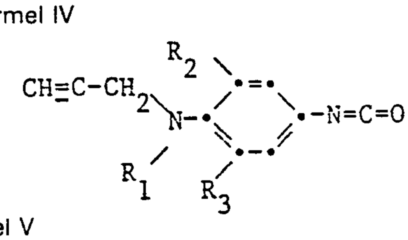
(IV)

mit einer Verbindung der Formel V

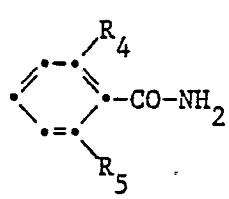
(V)

oder

c) einer Verbindung der Formel II mit einer Verbindung der Formel VI

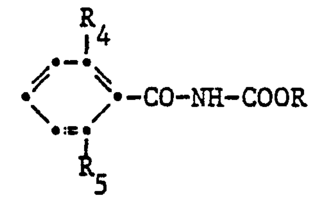

(VI)

In den obigen Formeln II, III, IV, V und VI haben die Reste $R_1$ bis $R_5$ die unter Formel I vorstehend angegebenen Bedeutungen und R bedeutet einen $C_1$—$C_8$-Alkylrest, der gegebenenfalls mit Halogen substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril;

Dimethylsulfoxid sowie Ketone, z.B. Aceton, Methyläthylketon, Methylisopropylketon und Methylisobutylketon. Verfahren a) wird im allgemeinen bei einer Tempertur von −10 bis 100°C, vorzugsweise zwischen 15 und 25°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit einer Verbindung der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylol, Chlorbenzol usw., verwendet werden.

Die Ausgangstoffe der vorstehenden Formeln III, V und VI sind bekannt oder lassen sich, falls sie neu sind, analog bekannten Verfahren herstellen.

Die Verbindungen der Formel II sind neu und bilden als Ausgangsverbindungen, welche zu den wertvollen Schädlingsbekämpfungsmitteln der Formel I führen, ebenfalls einen Gegenstand der vorliegenden Erfindung. Sie können z.B. durch chemische Reduktion entsprechender substituierter Nitrobenzole der Formel VII

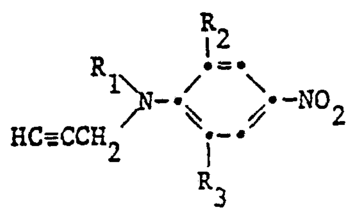

(VII)

mittels Eisen in wässrigen Säuren hergestellt werden. Die substituierten Nitrobenzole der Formel VII werden ihrerseits durch N-Propargylierung entsprechender Nitroverbindungen der Formel VIII

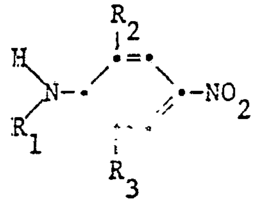

(VIII)

z.B. in Analogie zur im US-Patent 3.121.745 beschriebenen Arbeitsweise hergestellt. Die Isocyanate der Formel IV sind z.B. durch Phosgenisierung der entsprechenden N,N-substituierten p-Phenylendiamine der Formel II nach allgemein üblichen Arbeitsweisen erhältlich. Zu den Benzamiden der Formel V und den

Benzoylisocyanaten der Formel III kann man wie folgt gelangen (vgl. J. Arg. Food Chem. 21(3), 348 bzw. 993; 1973):

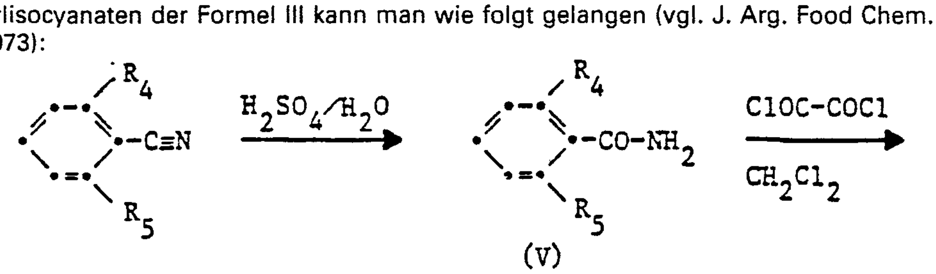

(V)
(III)

Die vorerwähnten als Ausgangsstoffe zu verwendenden Benzamide der Formel V sind zumeist bekannt (vgl. Beilstein "Handbuch der organischen Chemie" Bd. 9, S. 336). Die Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Es ist bereits bekannt, dass bestimmte N-Phenyl-N'-benzoylharnstoffe insektizide Eigenschaften besitzen (vgl. deutsche Offenlegungsschriften 2.123.236, 2.504.982, 2.537.413, 2.601.780 und 2.726.684, die belgischen Patentschriften 832.304, 743.906, 844.066 und 867.046 sowie die US-Patentschrift 4.089.975). Die deutsche Offenlegungsschrift 2.926.480 hat substituierte N-(p-Alkylenphenylamino)-phenyl-N'-benzoylharnstoffe und -thioharnstoffe mit insektizider Wirksamkeit zum Gegenstand. Aus J. Agr. Food Chem. *21*, No. 3, 348ff. (1973) sind weiterhin substituierte N-Phneyl-N'-2,6-dichlorbenzoylharnstoffe bekannt, die insektizide Eigenschaften aufweisen sollen. Auf Seite 353 dieser Veröffentlichung werden entsprechende N-(4-Dimethylamino)-phenyl- und N-(3-Chlor-4-dimethylamino)-phenylderivate erwähnt, die jedoch — wie aus der dort aufgeführten Tabelle III ersichtlich — nur unzureichende Insektizidwirkung zeigen. Die Europäische Patentanmeldung 16729 beschreibt substituierte N-(p-Alkenylamino)-phenyl-N'-benzoylharnstoffe, welche ebenfalls insektizide Eigenschaften besitzen.

Ueberraschenderweise wurde demgegenüber gefunden, dass die erfindungsgemässen Verbindungen der Formel I bei guter Pflanzenverträglichkeit und geringer Warmblütertoxizität höhere Wirksamkeit als Schädlingsbekämpfungsmittel aufweisen, als die vorerwähnten, aus dem Stand der Technik bekannten Verbindungen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer sehr günstigen Wirkung gegenüber Fliegen, wie z.B. Musca domestica, und Mückenlarven eignen sich Verbindungen der Formel I auch zur Bekämpfung von pflanzenschädigenden Frassinsekten, in Zier- und Nutzpflanzungen, insbesondere in Baumwollkulturen (z.B. Spodoptera littoralis und Heliothis virescens) sowie in Obst- und Gemüsekulturen (z.B. Leptinotarsa decemlineata, Pieris brassicae und Laspeyresia pomonella). Hervorzuheben ist besonders die ovizide bzw. ovolarvizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z.B. durch Tier-, Stall-oder Weidebehandlung.

Die gute insektizide Wirkung der erfindungsgemässen Verbindungen der Formel I entspricht einer Abtötungsrate (Mortalität) von mindestens 50—60% der erwähnten Schadinsekten.

Die Wirkung der erfindungsgemässen Verbindungen bzw. der sie enthaltenen Mittel lässt sich durch Zusatz von anderen Insektiziden und/oder Akariziden wesentlich verbreitern und an gegebene Umstände anpassen.

Als Zusätze kommen z.B. folgende Wirkstoff in Betracht.

organische Phosphorverbindungen,
Nitrophenol und Derivate,
Formamidine, Harnstoffe, Pyrethroide,
Carbamate,
chlorierte Kohlenwasserstoffe und Bacillus thuringiensis Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungsstechnik üblichen Stoffen, wie z.B. natürlichen oder regenerierten Stoffen, Lösungs-, Dispergier-, Netz-, Haft-, Verdickings-, Binde- und/oder Düngemitteln. Zur Applikation können die Verbindungen der Formel I zu Stäubemitteln, Emulsionskonzentrate, Granulaten, Dispersionen, Sprays, zu Lösungen oder Aufschlämmungen in üblicher Formulierung, die in der Applikationstechnik zum Allgemeinwissen gehören, verarbeitet werden. Ferner sind "cattle dips", d.h. Viehbäder, und "spray

races", d.h. Sprühgänge, in denen wässrige Zubereitung verwendet werden, zu erwähnen. Diese Zubereitungsformen sind insbesondere zur Bekämpfung tierparasitärer Schädlinge geeignet.

Die Verbindungen der Formel I werden in unveränderter Form oder vorzugsweise zusammen mit den in der Formulierungsstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I, bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie s.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Ataapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionigene, kation- und/oder anionaktive Tenside mit guten Emulgier-, Dispergier- und Netzeigenschaften in Betracht. Unter Tensiden sind auch Tensidgemische zu verstehen.

Geeignet anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erd-alkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. des Phosphorsäureesters eines p-Nonylphenol-(4-14)-Aethylenoxyd-Adduktes in Frage.

Als nichtionische Tenside kommen in erster Linie Propylglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkyl-rest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusöpolyglykoläther, Polypropylen-Polyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als

N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxy-alkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publicing Corp., Ringwood, New Jersey, 1979.

Sisely and Wood, "Encyclopedia of Surface Active Agents", Chemical Publishing Co., Inc. New York.

Die pestiziden Zubereitungen enthalten in der Regel 0,1 bis 99%, insbesondere 01 bis 95%, Wirkstoff der Formel I oder Kombinationen dieser Wirkstoffe mit andern Insektiziden oder Akariziden, 1 bis 99,9% eines festen oder flüssigen Zusatztoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentration aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

*Formulierungsbeispiele für flüssige Wirkstoffe der Formel I oder Kombinationen dieser Wirkstoffe mit andern Insektiziden oder Akariziden (% = Gewichtsprozent)*

| 1. *Emulsionskonzentrate* | a) | b) | c) |
|---|---|---|---|
| Wirkstoff resp. Wirkstoffkombination | 20% | 40% | 50% |
| Ca-Dodecylbenzolsulfonat | 5% | 8% | 5,8% |
| Ricinusölpolyäthylenglykoläther (36 Mol AeO) | 5% | — | — |
| Tributylphenolpolyäthylenglykoläther (30 Mol AeO) | — | 12% | 4,2% |
| Cyclohexanon | — | 15% | 20% |
| Xylolgemisch | 70% | 25% | 20% |

Aus solchen Konzentraten können durch Verdünnen mit Wasser Emulsionen jeder gewünschten Konzentrationen hergestellt werden.

| 2. *Lösungen* | | | | d) |
|---|---|---|---|---|
| Wirkstoff resp. Wirkstoffkombinationen | 80% | 10% | 5% | 95% |
| Aethylenglykolmonomethyläther | 20% | — | — | — |
| Polyäthylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

die Lösungen sind zur Andwendung in Form kleinster Tropfen geeignet.

| 3. *Granulate* | a) | b) |
|---|---|---|
| Wirkstoff resp. Wirkstoffkombination | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

4. *Stäubemittel*

| | | |
|---|---|---|
| Wirkstoff resp. Wirkstoffkombination | 2% | 5% |
| Hockdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemittel.

*Formulierungsbeispiele für feste Wirkstoffe der Formel I resp. Kombinationen dieser Wirkstoffe mit andern Insektiziden oder Akariziden (% = Gewichtsprozent)*

| 5. *Spritzpulver* | a) | b) |
|---|---|---|
| Wirkstoff oder Wirkstoffkombination | 20% | 60% |
| Na-Ligninsulfonat | 5% | 5% |
| Na-Laurylsulfat | 3% | — |
| Na-Diisobutylnaphthalinsulfonat | — | 6% |
| Octylphenolpolyäthylenglykoläther (7—8 Mol AeO) | — | 2% |
| Hochdisperse Kieselsäure | 5% | 27% |
| Kaolin | 67% | — |

Der Wirkstoff oder die Wirkstoffkombination werden it den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. *Emulsions-Konzentrat*

| | |
|---|---|
| Wirkstoff oder Wirkstoffkombination | 10% |
| Octylphenolpolyäthylenglykoläther (4—5 Mol AeO) | 3% |
| Ca-Dodecylbenzolsulfonat | 3% |
| Ricinusölpolyglykoläher (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. *Stäubemittel* | a) | b) |
|---|---|---|
| Wirkstoff oder Wirkstoffkombination | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

8. *Extruder-Granulat*

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

9. *Umhüllungs-Granulat*

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. *Suspensions-Konzentrat*

| | |
|---|---|
| Wirkstoff oder Wirkstoffkombination | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0.8% |
| Wasser | 32% |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

*a) Herstellung von 3,5-Dichlor-4-(N-methyl-N-propargyl)aminoanilin*

33,15 g 3,5-Dichlor-4-N-methyl-amino-nitrobenzol, 20 g gepulvertes NaOH, 24 g Propargylbromid und 3,8 g Tetrabutylammoniumhydrogensulfat in 250 ml Toluol werden 15 Stunden bei 20°C gerührt und anschliessend 4 Stunden auf 60°C erhitzt.

Nach dem Abkühlen wird filtriert und das Filtrat nach dem Entfernen des Lösungsmittels über Kieselgel mit Hexan-Diäthyläther (10:1) als Eluiermittel chromatographiert.

Nach dem Umkristallisieren aus Hexan erhält man die Verbindung der Formel

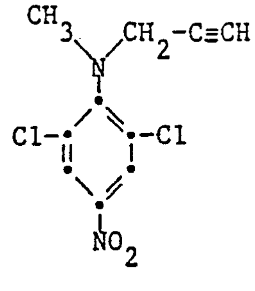

mit einem Schmelzpunkt von 54—56°C.

13,4 g 3,5-Dichlor-4-(N-methyl-N-propargyl)-amino-nitrobenzol werden in 50 ml Tetrahydrofuran und 200 ml 5%iger Essigsäure vorgelegt und bei Rückflusstemperatur portionenweise mit 45 g Eisenpulver versetzt. Nach beendeter Zugabe wird abgekühlt, filtriert und das Filtrat dreimal mit Diäthyläther extrahiert. Nach dem Entfernen der Lösungsmittel wird das Rohprodukt über Kieselgel mit Hexan-Diäthyläther (10:1) als Eluiermittel chromatographiert. Man erhält die Verbindung der Formel

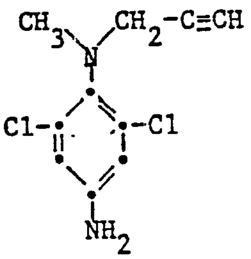

als gelbliches Oel.

*b) Herstellung von N-[3,5-Dichlor-4-(N-methyl-N-propargyl)amino]-phenyl-N'-2-chlorbenzoylharnstoff*

3,7 g 3,5-Dichlor-4-(N-methyl-N-propargyl)amino-anilin werden in wenig wasserfreiem Aether gelöst und unter Kühlen und Feuchtigkeitsausschluss mit 2,9 g 2-Chlorbenzoylisocyanat versetzt. Der Niederschlag wird abgesaugt und aus Alkohol umkristallisiert. Man erhält die Verbindung der Formel

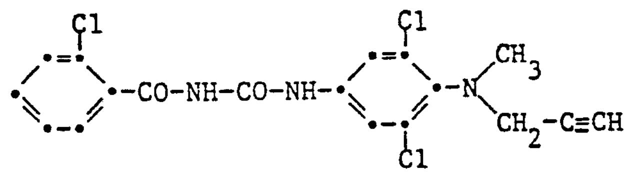

mit einem Schmelzpunkt von 138—140°C (Verbindung Nr. 1).

Analog der vorstehend beschriebenen Arbeitsweise wurden folgende Verbindungen der Formel I hergestellt:

| Verb. No. | $R_1$ | $R_2$ | $R_3$ | $R_4$ | $R_5$ | Schmelzpunkt (°C) |
|---|---|---|---|---|---|---|
| 2 | $—CH_3$ | Cl | Cl | Cl | H | 138—140 |
| 3 | $—CH_3$ | Cl | Cl | F | H | 156,5—159 |
| 4 | $—CH_3$ | Cl | Cl | Cl | F | 168—169 |
| 5 | $—C_2H_5$ | Cl | Cl | F | F | 133—134 |
| 6 | $—CH_2{\equiv}CH$ | Cl | Cl | F | F | 155—156 |
| 7 | $—CH_3$ | Cl | Br | F | F | 179—180,5 |
| 8 | $—CH_3$ | Cl | Br | Cl | H | 120—122 |
| 9 | $—CH_3$ | Cl | Br | F | H | 162—164,5 |
| 10 | $—C_2H_5$ | Cl | Cl | F | H | 127—128 |
| 11 | $—C_2H_5$ | Cl | Cl | Cl | H | 150—151 |
| 12 | $—(CH_2)_3CH_3$ | Cl | Cl | Cl | H | 159—161 |
| 13 | $—(CH_2)_3CH_3$ | Cl | Cl | F | H | [illegible] |
| 14 | $—(CH_2)_3CH_3$ | Cl | Cl | F | F | 176—179 |
| 15 | $—(CH_2)_2CH_3$ | Cl | Cl | Cl | F | 178—180 |
| 16 | $—(CH_2)_2CH_3$ | Cl | Cl | Cl | H | 134—136 |
| 17 | $—(CH_2)_2CH_3$ | Cl | Br | F | H | 90—92 |
| 18 | $—(CH_2)_2CH_3$ | Cl | Br | Cl | H | 168—170 |
| 19 | $—(CH_2)_2CH_3$ | Cl | Br | F | F | 178—181 |

Gemäss den vorstehend beschriebenen Arbeitsweisen sind auch die folgenden Verbindungen der Formel I erhältlich:

| Verb. No. | $R_1$ | $R_2$ | $R_3$ | $R_4$ | $R_5$ |
|---|---|---|---|---|---|
| 20 | $-CH_3$ | Cl | Cl | Br | H |
| 21 | $-CH_3$ | Cl | Cl | $CH_3$ | H |
| 22 | $-CH_3$ | F | Cl | F | F |
| 23 | $-CH_3$ | F | Cl | Cl | H |
| 24 | $-CH_3$ | Br | F | Cl | H |
| 25 | $-CH_3$ | Br | F | F | F |
| 26 | $-CH_3$ | Cl | F | F | H |
| 27 | $-(CH_2)_2CH_3$ | Cl | Cl | F | H |
| 28 | $-(CH_2)_3CH_3$ | Cl | Cl | F | F |
| 29 | $-CH_2-C\equiv CH$ | Cl | Cl | Cl | H |
| 30 | $-CH_2-C\equiv CH$ | Cl | Cl | F | Cl |
| 31 | H | Cl | Cl | F | F |

Beispiel 2

*Wirkung gegen Lucilia sericata*

Zu 9 ml eines Suchtmediums wurde bei 50°C 1 ml einer 0,5% Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeigen in diesem Test gute Wirkung gegen Lucilia sericata.

Beispiel 3

*Wirkung gegen Aëdes aegypti*

Auf die Oberfläche von 150 ml Wasser, das sich in einem Behälter befindet, wurde so viel einer 0,1%igen acetonischen Lösung des Wirkstoffes pipettiert, dass Konzentrationen von je 10, 5 und 1 ppm erhalten wurden. Nach Verdunsten des Acetons wurde der Behälter mit 30 bis 40 3-tägigen Aëdes-Larven beschickt. Nach 1, 2 und 5 Tagen wurde die Mortalität geprüft.

Verbindungen gemäss Beispiel 1 zeigen in diesem Test gute Wirkung gegen Aëdes aegypti.

Beispiel 4

*Insektizide Frassgift-Wirkung*

Ca. 25 cm hohe eingetopfte Baumwollpflanzen wurden mit wässrigen Wirkstoffemusionen besprüht, die den Wirkstoff in Konzentrationen von 800, 400, 200, 100, 50, 12,5 und 3,0 ppm enthielten.

Nach dem Antrocknen des Sprühbelages wurden die Baumwollpflanzen mit Spodoptera littoralis- bzw. Heliothis virescens-Larven im dritten larvalen Stadium besiedelt. Der Versuch wurde bei 24°C und 60% relativer Luftfeuchtigkeit durchgeführt. Nach 120 Stunden wurde die %-Mortalität der Test-Insekten ausgewertet.

Die nachstehende Tabelle zeigt Ergebnisse biologischer Prüfungen erfindungsgemässer Verbindungen auf der Basis des obigen biologischen Beispiels. Die Auswertung der Versuche erfolgte anhand der erhaltenen %-Mortalität unter Verwendung des folgenden Bewertungs-Index:

A: 80—100% Mortalität bei einer Konzentration von 3,0 ppm der geprüften Verbindung
B: 80—100% Mortalität bei einer Konzentration von 12,5 ppm der geprüften Verbindung
C: 80—100% Mortalität bei einer Konzentration von 50 ppm der geprüften Verbindung
D: 80—100% Mortalität bei einer Konzentration von 100 ppm der geprüften Verbindung
E: 80—100% Mortalität bei einer Konzentration von 200 ppm der geprüften Verbindung
F: 80—100% Mortalität bei einer Konzentration von 400 ppm der geprüften Verbindung
G: weniger als 80% Mortalität bei einer Konzentration von 800 ppm der geprüften Verbindung

| Verbindung Nr. | pestizide Wirksamkeit | |
|---|---|---|
| | Spodoptera Larven (Beispiel 4) | Heliothis Larven (Beispiel 4) |
| 1 | A | B |
| 2 | B | C |
| 3 | C | D |
| 4 | A | C |
| 5 | B | B |
| 6 | A | C |
| 7 | B | C |
| 8 | A | B |
| 9 | F | G |
| 10 | B | E |

Beispiel 5

*Ovizide Wirkung auf Spodoptera littoralis*

Auf Filterpapier abgelegte Eier von Spodoptera littoralis, die nicht älter als 24 Stunden waren, wurden aus dem Papier ausgeschnitten und in eine Lösung von 400 ppm des Wirkstoffes in einem Aceton-Wasser-Gemisch (1:1) getaucht. Die Eintauchzeit betrug eine Minute. Die so behandelten Eiablagen wurden dann aus der Lösung herausgenommen und bei 28°C und 60% relativer Feuchtigkeit in Kunststoffschalen deponiert.

Nach 5 Tagen wurde die Schlupfrate, d.h. die Anzahl Larven, die sich aus den behandelten Eiern entwickelt hatten, bestimmt.

Verbindungen gemäss Beispiel 1 zeigen gute Wirkung im obigen Test.

Beispiel 6

*Wirkung auf Lasperyresia pomonella (Eier)*

Abgelegte Eier von Laspeyresia pomonella, die nicht älter als 24 Stunden waren, wurden auf Filterpapier für 1 Minute in eine acetonischwässrige Lösung, enthaltend 12,5 ppm des zu prüfenden Wirkstoffes, eingetaucht. Nach dem Antrocknen der Lösung wurden die Eier in Petrischalen ausgelegt und bei einer Temperatur von 28°C belassen. Nach 6 Tagen wurde der prozentuale Schlupf aus den behandelten Eiern bewertet.

Die Verbindungen Nr. 4 und 5 gemäss Beispiel 1 zeigten in obigem Test eine 100%-ige Wirkung. Andere Verbindungen gemäss Beispiel 1 erwiesen sich in obigem Test ebenfalls als gut wirksam.

Beispiel 7

*Reproduktions-Beeinflussung von Anthonomus grandis*

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.% des zu prüfenden Wirkstoffes getaucht.

Nachdem die Käfer wieder trocken waren, wurden sie zur Kopulation und Eiablage in angedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z.B. "Actamer B 100") desinfiziert und dann in Schalen, die eine geeignete Larvaldiät enthielten, deponiert. Nach 7 Tagen wurde untersucht, ob sich aus den deponierten Eiern Larven entwickelt hatten.

Sur Ermittlung der Dauer eines die Reproduktion beeinflussenden Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und der daraus geschlüpften Larven im Vergleich zu unbehandelten Kontrollen.

Verbindungen gemäss Beispiel 1 zeigen eine gute, die Reproduktion reduzierende Wirkung im obigen Test.

**Patentansprüche**

1. Verbindung der Formel I

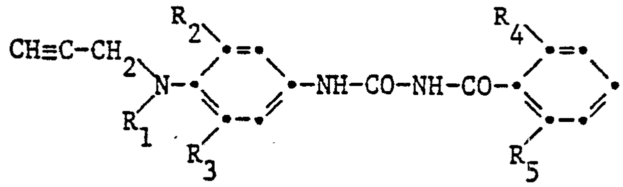

(I),

worin $R_1$ Wasserstoff, $C_1$—$C_4$-Alkyl oder Propargyl; $R_2$ und $R_3$ je Wassertoff oder Halogen; $R_4$ Methyl oder Halogen und $R_5$ Wasserstoff oder Halogen bedeuten.

2. Verbindung der formel I gemäss Anspruch 1, dadurch gekennzeichnet, dass $R_1$ $C_1$—$C_4$-Alkyl oder Propargyl; $R_2$ und $R_3$ Fluor, Chlor oder Brom; $R_4$ Fluor, Chlor, Brom oder Methyl und $R_5$ Wasserstoff, Fluor oder Chlor bedeuten.

3. Verbindung der Formel I gemäss Anspruch 2, dadurch gekennzeichnet, dass $R_4$ Fluor oder Chlor bedeutet.

4. Verbindung der Formel I gemäss Anspruch 2, dadurch gekennzeichnet, dass $R_5$ Fluor oder Chlor bedeutet.

5. Verbindung der Formel I gemäss Anspruch 1, dadurch gekennzeichnet, dass $R_1$ $C_1$—$C_4$-Alkyl oder Propargyl; $R_2$ und $R_3$ Fluor, Chlor oder Brom; $R_4$ Fluor oder Chlor und $R_5$ Wasserstoff oder Fluor bedeuten.

6. Verbindung der Formel I gemäss Anspruch 5, dadurch gekennzeichnet, dass $R_4$ und $R_5$ Fluor bedeuten.

7. Verbindung der Formel I gemäss Anspruch 5, dadurch gekennzeichnet, dass $R_5$ Wasserstoff bedeutet.

8. Verbindung gemäss Anspruch 6 der Formel

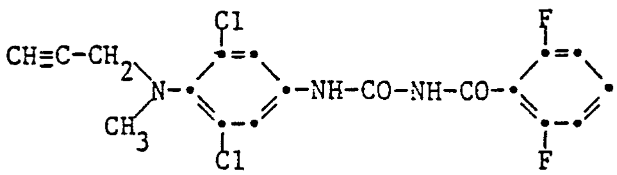

9. Verbindung gemäss Anspruch 5 der Formel

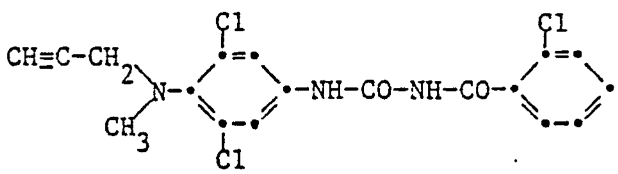

10. Verbindung gemäss Anspruch 5 der Formel

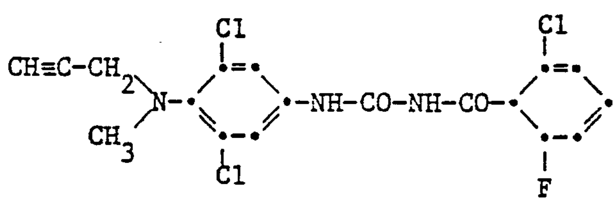

11. Verbindung gemäss Anspruch 6 der Formel

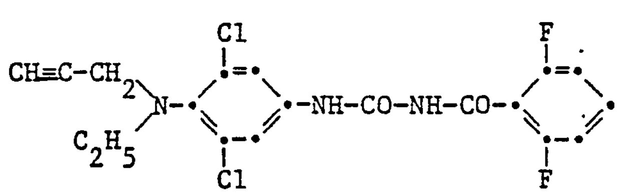

12. Verbindung gemäss Anspruch 6 der Formel

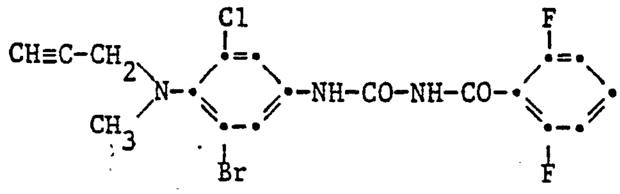

13. Vebindung gemäss Anspruch 5 der Formel

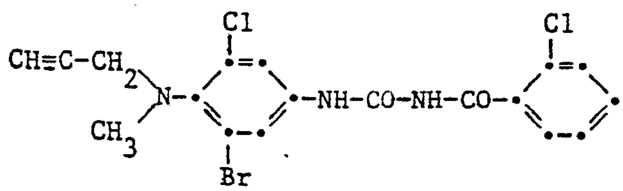

14. Verbindung gemäss Anspruch 6 der Formel

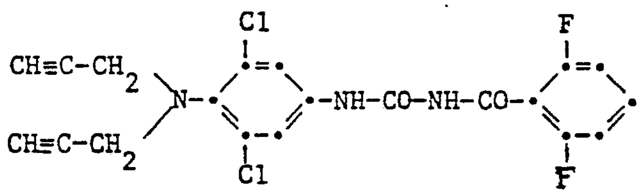

15. Verfahren zur Herstellung einer Verbindung der Formel I gemäss Anspruch 1 bis 14, dadurch gekennzeichnet, dass man

a) eine Verbindung der Formel II

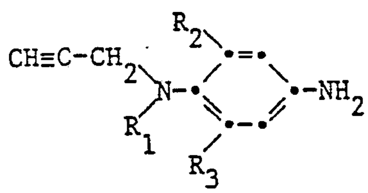

(II)

mit einer Verbindung der Formel III

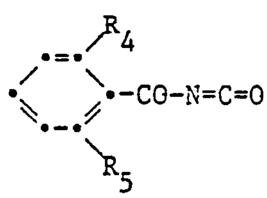

(III)

oder

b) eine Verbindung der Formel IV

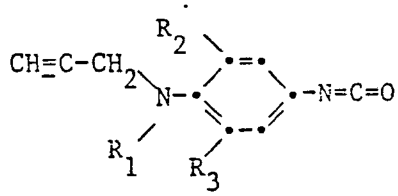

(IV)

mit einer Verbindung der Formel V

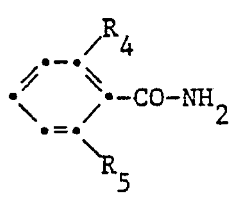

(V)

oder

c) ein Verbindung der Formel II mit einer Verbindung der Formel VI

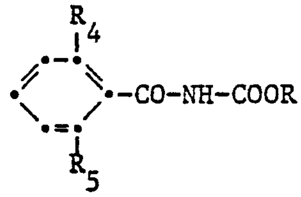

(VI)

umsetzt, wobei in den Formeln II bis VI die Reste $R_1$ bis $R_5$ die in den Ansprüchen 1 bis 7 angegebenen Bedeutungen haben und R für einen $C_1$—$C_8$-Alkylrest, der gegebenenfalls mit Halogen substituiert ist, steht.

16. Schädlingsbekämpfungsmittel, welches als aktive Komponente eine Verbindung gemäss Anspruch 1 bis 14, zusammen mit geeigneten Träger-und/oder anderen Zuschlagstoffen enthält.

17. Verwendung einer Verbindung gemäss Anspruch 1 bis 14 zur Bekämpfung von Schädlingen, vorzugsweise von Insekten, insbesondere von pflanzenschädigenden Insekten.

18. Verbindung gemäss Formel II

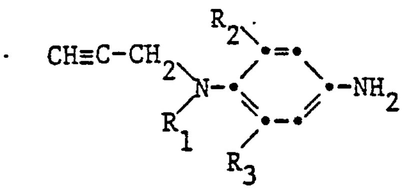

(II)

worin $R_1$ Wasserstoff, $C_1$—$C_4$-Alkyl oder Propargyl und $R_2$ und $R_3$ je Wasserstoff oder Halogen bedeuten.

**Claims**

1. A compound of the formula I

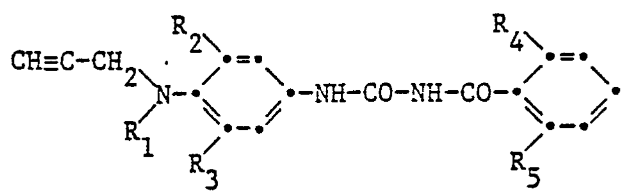

(I),

wherein $R_1$ is hydrogen, $C_1$—$C_4$ alkyl or propargyl; $R_2$ and $R_3$ are each hydrogen or halogen; $R_4$ is methyl or halogen; and $R_5$ is hydrogen or halogen.

2. A compound of the formula I according to claim 1, wherein $R_1$ is $C_1$—$C_4$ alkyl or propargyl; $R_2$ and $R_3$ are each fluorine, chlorine or bromine; $R_4$ is fluorine, chlorine, bromine or methyl; and $R_5$ is hydrogen, fluorine or chlorine.

3. A compound of the formula I according to claim 2, wherein $R_4$ is fluorine or chlorine.

4. A compound of the formula I according to claim 2, wherein $R_5$ is fluorine or chlorine.

5. A compound of the formula I according to claim 1, wherein $R_1$ is $C_1$—$C_4$ alkyl or propargyl; $R_2$ and $R_3$ are each fluorine, chlorine or bromine; $R_4$ is fluorine or chlorine; and $R_5$ is hydrogen or fluorine.

6. A compound of the formula I according to claim 5, wherein $R_4$ and $R_5$ are each fluorine.

7. A compound of the formula I according to claim 5, wherein $R_5$ is hydrogen.

8. A compound according to claim 6 of the formula

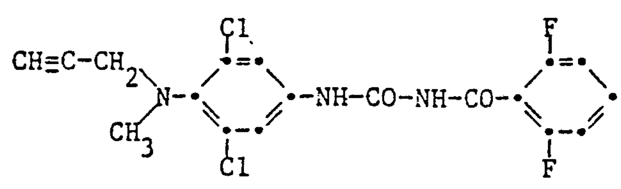

9. A compound according to claim 5 of the formula

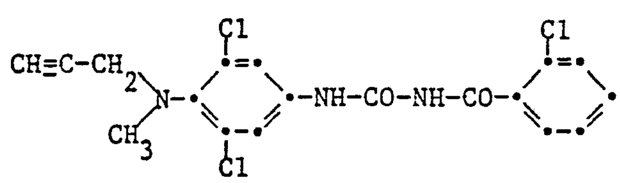

10. A compound according to claim 5 of the formula

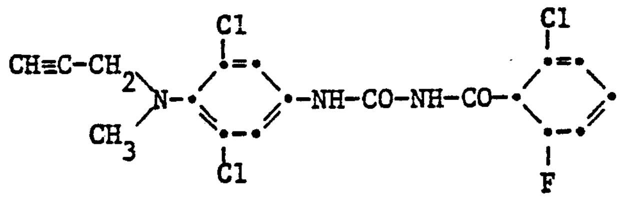

.

11. A compound according to claim 6 of the formula

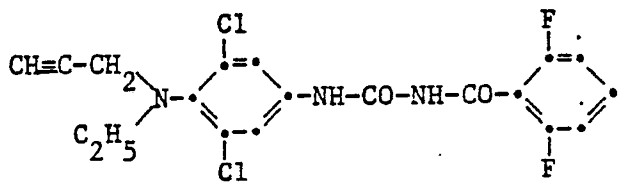

.

12. A compound according to claim 6 of the formula

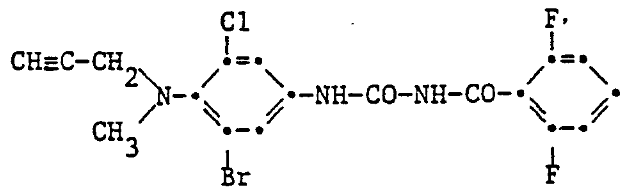

13. A compound according to claim 5 of the formula

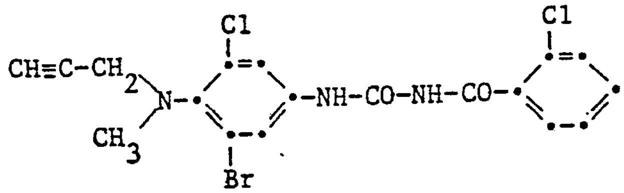

.

14. A compound according to claim 6 of the formula

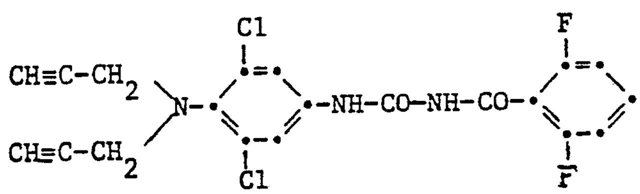

.

15. A process for the preparation of a compound of the formula I according to any one of claims 1 to 14, which process comprises reacting
a) a compound of the formula II

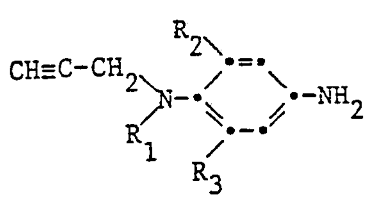

(II)

with a compound of the formula III

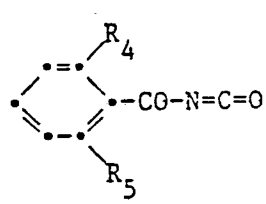

(III)

or

b) a compound of the formula IV

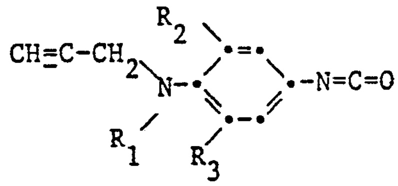

(IV)

with a compound of the formula V

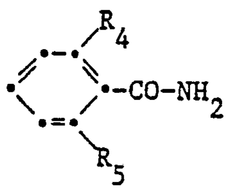

(V)

or

c) a compound of the formula II with a compound of the formula VI

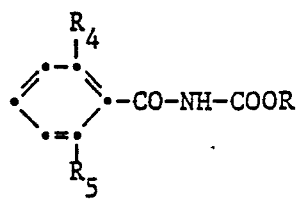

(VI)

the radicals $R_1$ to $R_5$ in the formulae II to VI being as defined in any one of claims 1 to 7, and R being a $C_1$—$C_8$-alkyl group which is unsubstituted or substituted by halogen.

16. A pesticidal composition which contains as active ingredient a compound according to any one of claims 1 to 14, together with suitable carriers and/or other additives.

17. A method of controlling pests, preferably insects, especially plant-destructive insects, which method comprises applying to said pests or to the locus thereof a pesticidally effective amount of a compound according to any one of claims 1 to 14.

18. A compound according to the formula II

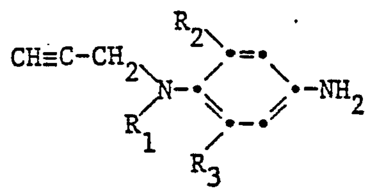

(II)

wherein $R_1$ is hydrogen $C_1$—$C_4$ alkyl or propargyl, and $R_2$ and $R_3$ are each hydrogen or halogen.

**Revendications**

1. Composé de formule I

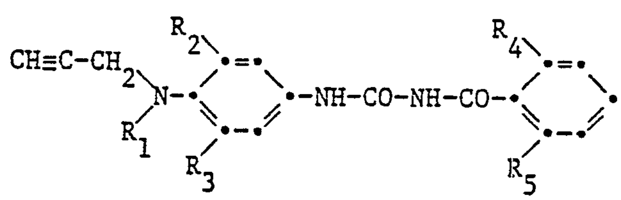

(I),

dans laquelle $R_1$ représente l'hydrogène, un groupe alkyle en $C_1$—$C_4$ ou propargyle; $R_2$ et $R_3$ représentent chacun l'hydrogène ou un halogène; $R_4$ représente un groupe méthyle ou un halogène et $R_5$ l'hydrogène ou un halogène.

2. Composé de formule I selon la revendication 1, caractérisé en ce que $R_1$ représente un groupe alkyle en $C_1$—$C_4$ ou propargyle; $R_2$ et $R_3$ représentent le fluor, le chlore ou le brome; $R_4$ représente le fluor, le chlore, le brome ou un groupe méthyle et $R_5$ l'hydrogène, le fluor ou le chlore.

3. Composé de formule I selon la revendication 2, caractérisé en ce que $R_4$ représente le fluor ou le chlore.

4. Composé de formule I selon la revendication 2, caractérisé en ce que $R_5$ représente le fluor ou le chlore.

5. Composé de formule I selon la revendication 1, caractérisé en ce que $R_1$ représente un groupe alkyle en $C_1$—$C_4$ ou propargyle; $R_2$ et $R_3$ représentent le fluor, le chlore ou le brome; $R_4$ représente le fluor ou le chlore et $R_5$ l'hydrogène ou le fluor.

6. Composé de formule I selon la revendication 5, caractérisé en ce que $R_4$ et $R_5$ représentent le fluor.

7. Composé de formule I selon la revendication 5, caractérisé en ce que $R_5$ représente l'hydrogène.

8. Composé selon la revendication 6 de formule:

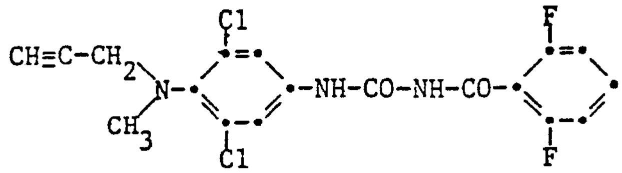

9. Composé selon la revendication 5 de formule:

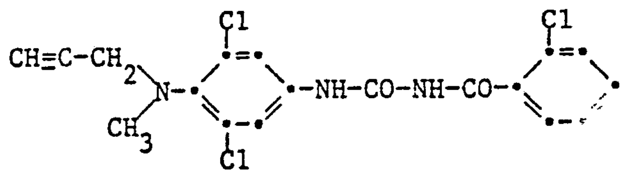

10. Composé selon la revendication 5 de formule:

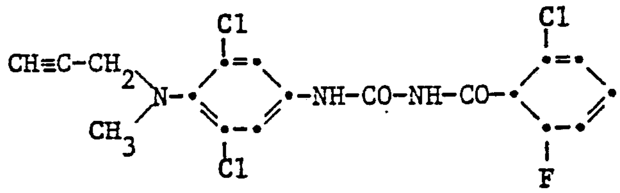

11. Composé selon la revendication 6 de formule:

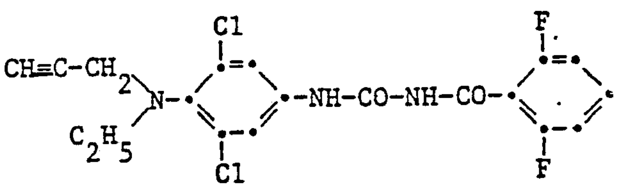

12. Composé selon la revendication 6 de formule:

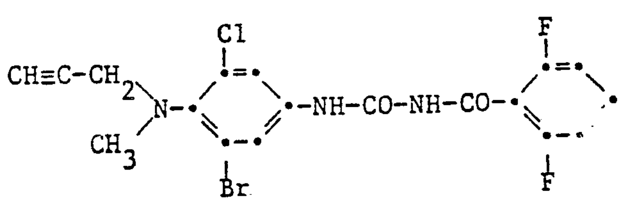

13. Composé selon la revendication 5 de formule:

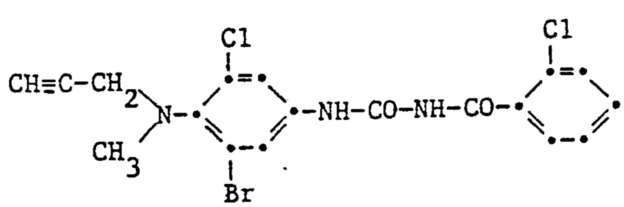

14. Composé selon la revendication 6 de formule:

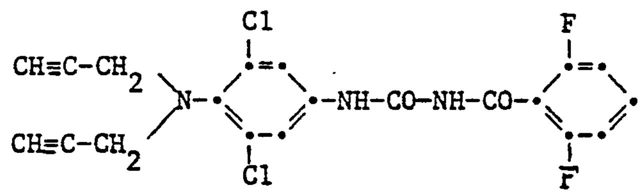

15. Procédé de préparation d'un composé de formule I selon les revendications 1 à 14, caractérisé en ce que l'on fait réagir:

a) un composé de formule II:

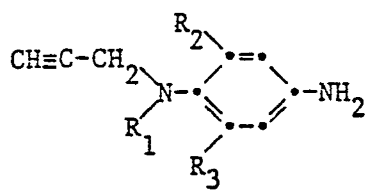

(II)

avec un composé de formule III:

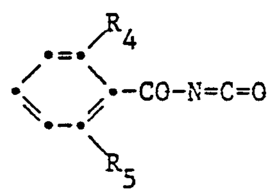

(III)

ou bien

b) un composé de formule IV:

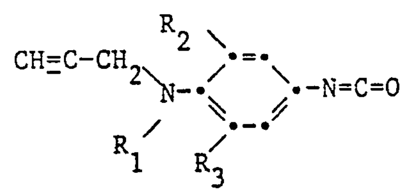

(IV)

avec un composé de formule V:

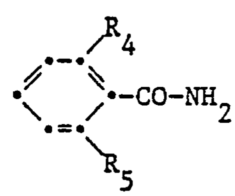

(V)

ou bien

c) un composé de formule II avec un composé de formule VI

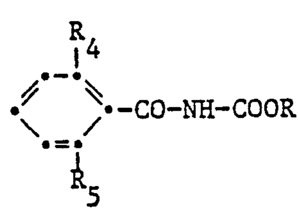

(VI)

les symboles $R_1$ à $R_5$ ayant dans les formules II à VI les significations indiquées dans les revendications 1 à 7 et R représentant un groupe alkyle en $C_1$—$C_8$ éventuellement substitué par des halogènes.

16. Produit pesticide contenant en tant que composant actif un composé selon les revendications 1 à 14 avec des véhicules et/ou d'autres additifs appropriés.

17. Utilisation d'un composé selon les revendications 1 à 14 dans le lutte contre les parasites, de préférence les insectes, en particulier les insectes nuisibles pour les végétaux.

18. Composé de formule II

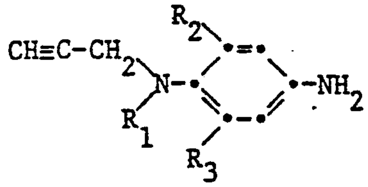

(II)

dans laquelle $R_1$ représente l'hydrogène, un groupe alkyle en $C_1$—$C_4$ ou propargyle et $R_2$ et $R_3$ représentent chacun l'hydrogène ou un halogène.